# EUROPEAN PATENT APPLICATION

(11) **EP 4 180 013 A1**
(43) Date of publication of application: **17.05.2023**
(21) Application number: 21207950.3
(22) Date of filing: 12.11.2021
(51) Int. Cl.: A61F 13/15, C09J 5/00, B05C 1/00

(54) **METHOD FOR MANUFACTURING SANITARY ARTICLES, METHOD FOR APPLYING A GLUE PATTERN ON A WEB, AND RELATED APPARATUSES**

(71) Applicant: Fameccanica.Data S.p.A., 66020 San Giovanni Teatino (CH) (IT)
(72) Inventor: LUPINETTI, Serafino, 66020 San Giovanni Teatino (Chieti) (IT)
(74) Representative: Marchitelli, Mauro

(57) **Abstract**

Disclosed herein are a method for manufacturing sanitary articles, a method for applying a glue pattern, and related apparatuses. The invention allows application of a pattern of glue onto a support sheet provided for making parts of sanitary articles.

## Description

### Field of the invention

The present invention relates to manufacturing of sanitary articles, such as sanitary pads, panty liners, diapers, training pants, paper towels, female hygiene products, etc.

The invention also relates to a method and apparatus for applying a glue pattern onto a support web provided for making parts of sanitary articles.

The invention applies both to washable and disposable sanitary articles.

### Description of the prior art

In order to produce absorbent sanitary articles, some portions of the articles, such as elastic bands for the legs, elastic leg barriers, elastic side panels, elastic waistbands, etc., are made from elastic laminates which can be produced in various ways depending on the characteristics of the absorbent sanitary articles. In particular, some types of elastic laminates may be formed from one or more non-woven webs bonded to an elastic film and/or wires. In other cases, absorbent bodies have to be bonded to a continuous sheet.

In the known solutions for producing such portions of the articles, glue application units may be provided for applying glue areas onto a continuous sheet, such as onto the inner surface of an absorbent continuous composite web. Therefore, it may be envisaged the application of a pattern of glue which takes place by using glue applicators which intermittently applies strips of glue onto elements which has to be connected to each other.

In these solutions, it may result extremely critical to obtain a precise application of a specific glue pattern on the area that need to be glued, without wasting a significant amount of glue sticking out from the target area, such as the area of elastic bands for the legs of diapers. Moreover, the known solutions are not suitable for precisely obtaining glue spots with circular shape.

### Object and summary of the invention

The present invention aims to provide a method and an apparatus for applying a glue pattern on a web that overcomes the problems of the prior art.

According to the present invention, this object is achieved by a method and an apparatus having the features of claims 1 and 11.

According to another aspect, the invention relates to a method for manufacturing absorbent sanitary articles having the features of claim 14 and to an apparatus for manufacturing absorbent sanitary articles having the features of claim 15.

The claims form an integral part of the disclosure provided here in relation to the invention.

### Brief description of the figures

The present invention will now be described in detail with reference to the attached figures, even purely by way of non-limiting example, wherein:
- Figure 1 is a perspective view of a washable absorbent garment in an open configuration,
- Figure 2 is a plan view of the absorbent garment of figure 1 in the open configuration,
- Figure 3 is a schematic cross-section taken along the line III-III of figure 2,
- Figures 4 is a schematic side view of an apparatus for producing the washable absorbent garment of figures 1-3,
- Figures 5-7 are schematic plan views showing intermediate steps of a method for producing washable absorbent garments,
- Figure 8 is a schematic side view showing further parts of the apparatus shown in figure 4,
- Figure 9 is a schematic cross-sectional view showing a preferred embodiment of an apparatus for applying a glue pattern onto a support web, and
- Figures 10,11 are schematic cross-sectional views showing further embodiments of an apparatus for applying a glue pattern onto a support web.

It will be appreciated that in the figures some components may not be illustrated to simplify the understanding of the figures.

### Detailed description

The invention relates to a method and apparatus for applying a glue pattern onto a support web provided for making parts of sanitary articles. According to another aspect, the invention relates to an apparatus and method for manufacturing absorbent sanitary articles.

In the following description it is widely described the application for making washable and reusable absorbent garments. Naturally, the invention related to a method and apparatus for applying a glue pattern is applicable to other sanitary articles, such as disposable absorbent articles (sanitary pads panty liners, diapers, training pants, paper towels, female hygiene products, etc.)

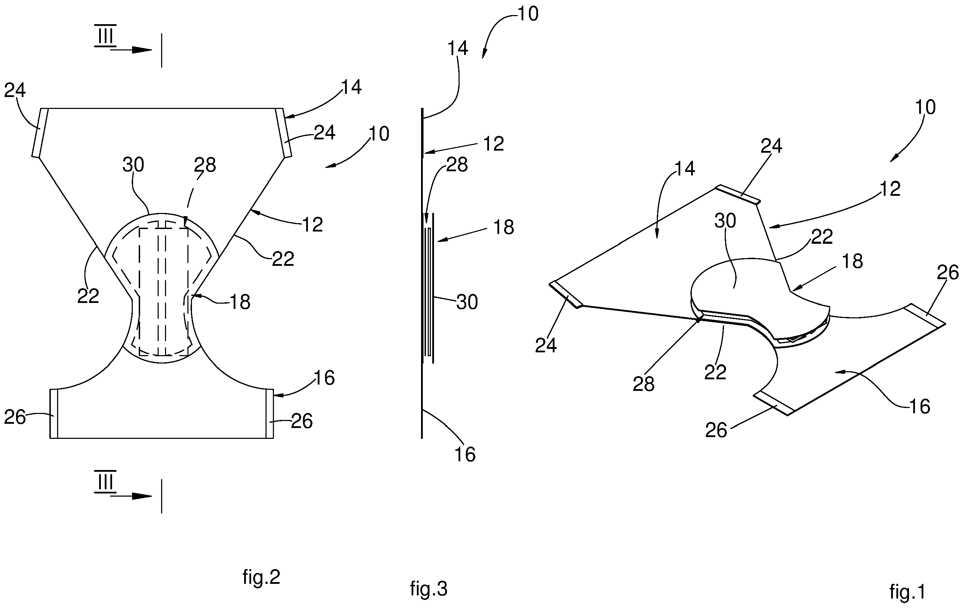

With reference to figures 1-3 the numeral reference 10 indicates a washable absorbent garment.

The washable absorbent garment 10 comprises a back layer 12 of washable fabric forming a pant structure. The back layer 12 has rear and front waist sections 14, 16 and a crotch section 18 intermediate between the rear and front waist sections 14, 16. The rear and front waist sections 14, 16 are wider than the crotch section 18, such that in the extended position of figures 1 and 2 the back layer 12 has substantially an hourglass shape.

The back layer 12 is a fabric formed by woven threads which is washable as ordinary textile garments, e.g. swimsuits, underwear, etc. The back layer 12 may be made of any natural or synthetic material used for producing textile articles, e.g. cotton, Lycra, nylon, polyester, etc. and any mixture thereof. In possible embodiments, the back layer 12 may be an elastic fabric made of a mixture of cotton and elastane, e.g. 80% cotton and 20% elastane or 80% PA (polyamide) and 20% EA (elastane). The back layer 20 may have a weight comprised between 150-200 g/m².

The crotch section 18 of the back layer 12 has two curved side edges 22 shaped to conform to the legs of the user in the configuration in which the washable absorbent garment 10 is worn. The rear and front waist sections 14, 16 of the back layer 12 have respective side edges 24, 26 which are joined to each other such that the washable absorbent garment 10 is closed in a pant-like shape and has two leg openings on opposite sides of the crotch section 18.

The side edges 24, 26 are joined to each other by glue and/or ultrasonic sealing.

The washable absorbent garment 10 comprises a washable absorbent core 28 permanently fixed in the crotch section 18 of the back layer 12. The washable absorbent core 28 extends only on the crotch section 18 of the back layer 12 and does not extend on the rear and front waist sections 16, 18 of the back layer 12.

The washable absorbent core 28 includes a washable absorbent pad comprising washable superabsorbent fibers. A material suitable for producing the washable absorbent pad 30 may be the one marketed by Technical Absorbents Ltd (UK) under the trade name SAF^{®}. The washable absorbent pad may be formed by needlefelt, thermal bonded, laminated non-woven fibers, formed by polyester, polypropylene and SAF^{®}. The washable absorbent pad may have a weight comprised between 100 and 400 g/m² and a thickness comprised between 1 and 4 mm.

The washable absorbent core 28 may include an acquisition and diffusion layer (ADL) and a barrier film. The washable absorbent pad may be sandwiched between the acquisition and diffusion layer and the barrier film.

The washable absorbent core 28 may have a total weight comprised between 150and 500 g/m² and a total thickness comprised between 1,5 and 5 mm.

The washable absorbent garment 10 comprises a top layer 30 of washable fabric which covers the washable absorbent core 28. The top layer 30 may be made of the same material as the fabric forming the back layer 12 or fabric textile 100 PP and/or other antiallergic or antibacterial textile. The top layer 30 completely covers the washable absorbent core 28 but does not extend on the rear and front waist sections 16, 18 of the back layer 12.

The top layer 30 and the washable absorbent core 28 are permanently joined to the back layer 12 by glue and/or ultrasonic sealing.

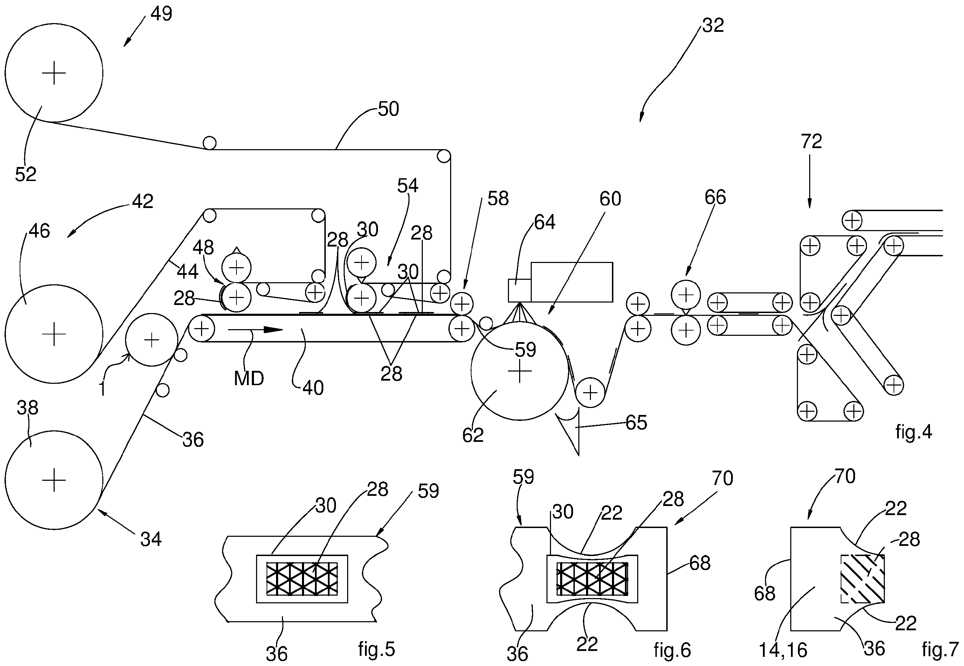

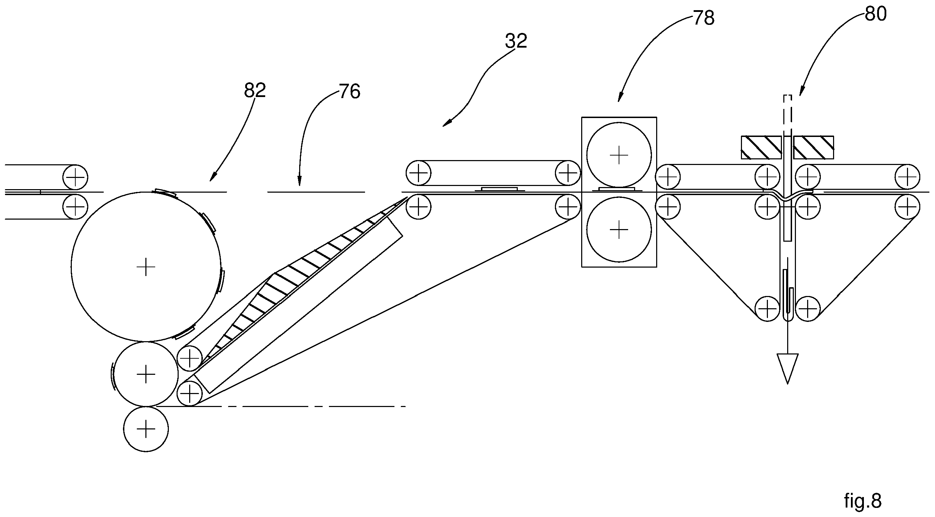

An embodiment of an apparatus for producing the washable absorbent garment 10 of the type illustrated in Figures 1-3 is shown in figures 4 and 8 and is indicated by the reference numeral 32.

With reference to figure 4, the apparatus 32 comprises a first unwinding unit 34 configured for unwinding a first continuous fabric layer 36 from a first reel 38. The first continuous fabric layer 36 is advanced in a machine direction MD on a conveyor 40.

The apparatus 32 comprises a second unwinding unit 42 configured for unwinding a continuous absorbent tape 44 from a second reel 46. The continuous absorbent tape 44 may comprise a web of superabsorbent fibers sandwiched between an acquisition and diffusion layer and a barrier layer. Alternatively, an acquisition and diffusion layer and a barrier layer may be applied in-line on opposite sides of a web of superabsorbent fibers unwound from the second reel 46.

The apparatus 32 comprises a first cut-and-slip unit 48 configured for transversally cutting the continuous absorbent tape 44 to form individual absorbent cores 28. The first cut-and-slip unit 48 is configured for longitudinally spacing from each other the individual absorbent cores 28 and for applying the individual absorbent cores 28 on the first continuous fabric layer 36 in longitudinally spaced positions on the conveyor 40.

The apparatus 32 comprises a third unwinding unit 49 configured for unwinding a second continuous fabric layer 50 from a third reel 52. The apparatus 32 comprises a second cut-and-slip unit 54 configured for transversally cutting the second continuous fabric layer 50 to form individual top layers 30. The second cut-and-slip unit 54 is configured for longitudinally spacing from each other the individual top layers 30 and for applying the individual top layers 30 on respective absorbent cores 28 previously applied on the first continuous fabric layer 36 on the conveyor 40.

The apparatus 32 comprises an apparatus 1 configured for applying a pattern of glue on the first continuous fabric layer 36 for permanently attaching by glue the individual absorbent cores 28 and the individual top layers 30 on the first continuous fabric layer 36.

The apparatus 1 for applying a pattern of glue is described more in detail in the following.

The apparatus 32 comprises a sealing press 58 located at the end of the conveyor 40, configured for compressing the individual absorbent cores 28 and the individual top layers 30 to the first continuous fabric layer 36. After the compression, the individual absorbent cores 28 and the individual top layers 30 are permanently fixed by glue to the first continuous fabric layer 36 in longitudinally spaced positions to form a continuous garment tape 59.

Figure 5 shows a portion of the continuous garment tape 59 formed by individual absorbent cores 28 and individual top layers 30 fixed to the first continuous fabric layer 36.

The apparatus 32 comprises a cutting unit 60 configured for cutting longitudinal side edges of the continuous garment tape 59 as it advances in the machine direction MD along curved profiles shaped to conform to the legs of the user. The cutting unit 60 comprises an anvil roller 62 and a cutting tool 64 cooperating with the outer surface of the anvil roller 62. The cutting tool 64 may be a laser nozzle or an ultrasonic horn configured for carrying out a laser or ultrasonic cut of the first continuous fabric layer 36. The cutting tool 64 may also cut simultaneously side edges of the top layer 30 and of the absorbent core 28.

The cutting tool 64 may be movable transversally to the machine direction to form, in conjunction with the longitudinal movement of the continuous garment tape 59, curved cuts which form curved side edges 22 (figure 7) of the continuous garment tape 59. The portions trimmed from the continuous garment tape 59 are collected in a waste collecting channel 65.

The apparatus 32 comprises a transverse cutting unit 66 configured to cut the first continuous fabric layer 36 along transverse cutting lines 68 (figure 6) to form individual blank absorbent garments 70. Figure 6 shows the continuous garment tape 59 after the longitudinal and transverse cutting steps.

The apparatus 32 comprises a transverse folding unit 72 configured for folding the individual blank absorbent garments 70 along respective transverse folding lines, so as to overlap to each other the rear and front waist sections 14, 16 of each blank absorbent garment 70. Figure 7 shows a blank absorbent garment 70 after the transverse folding step.

In the embodiment of figure 8, the side edges 24, 26 of the rear and front waist sections 14, 16 of the blank absorbent garments 70 are fixed to each other by glue. The apparatus 1 for applying a glue pattern (figures 4,9,10) is configured for applying discrete glue layers also on the portions of the first continuous fabric layer 36 which will form the side edges 24, 26 of the rear and front waist sections 14, 16 of the blank absorbent garments 70. In the embodiment of figure 8, the apparatus 32 comprises a press unit 82 which receives the folded blank absorbent garments 70 from the folding unit 72. The press unit 82 is configured to press the side edges 24, 26 of the rear and front waist sections 14, 16 for glue sealing the side edges 24, 26.

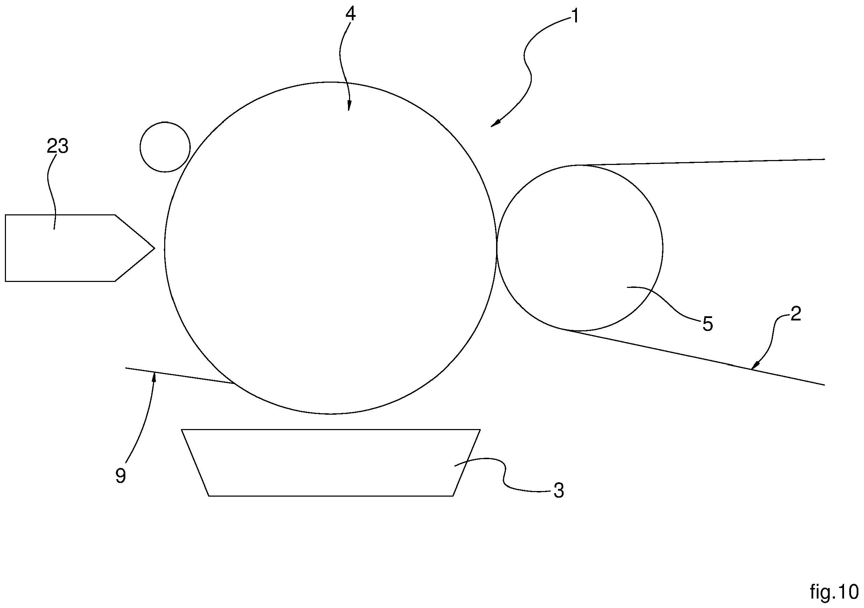

The apparatus 32 may comprise a linear folding unit 76 configured for folding side portions of the finished absorbent garments 10 as shown in figure 10.

The apparatus 32 may comprise a final press unit 78 configured for pressing the folded portions of the finished absorbent garments 10.

The apparatus 32 may comprise a final folding unit 80 configured for carrying out a final folding of the finished absorbent garments 10. After the final folding unit 80 the finished absorbent garments 10 may be sent to a packaging machine.

In operation, the apparatus 32 implements a method for producing washable absorbent garments 10, comprising:
- providing a first continuous fabric layer 36 movable in a longitudinal direction MD,
- applying discrete glue layers on said first continuous fabric layer 36,
- providing a continuous washable absorbent tape 44 containing washable superabsorbent fibers, continuously moving in a longitudinal direction,
- transversely cutting said continuous washable absorbent tape 44 to form individual washable absorbent cores 28,
- spacing from each other in a longitudinal direction said individual washable absorbent cores 28,
- applying said washable absorbent cores 28 on said first continuous fabric layer 36 in longitudinally spaced positions over respective glue layers,
- providing a second continuous fabric layer 50 continuously moving in a longitudinal direction,
- transversely cutting said second continuous fabric layer 50 to form individual top layers 30,
- spacing from each other in a longitudinal direction said individual top layers 30,
- applying said individual top layers 30 over respective washable absorbent cores 28 on said first continuous fabric layer 36, and
- joining said washable absorbent cores 28 and said individual top layers 30 to said first continuous fabric layer 36 by glue to form a continuous washable garment tape 59, and
- transversely cutting said continuous washable garment tape 59 to form individual blank absorbent garments 70.

The method and apparatus according to the present invention may have a production rate up to 200 pieces/1', which is a huge progress as compared to the production rate of prior art washable absorbent products which is about 1 piece/1'.

As previously indicated, an apparatus 1 is configured for applying a pattern of glue on a support web for making absorbent sanitary articles. The apparatus 1 is applicable to different types of sanitary articles, such as disposable absorbent articles (sanitary pads panty liners, diapers, training pants, paper towels, female hygiene products, etc.) and washable sanitary articles.

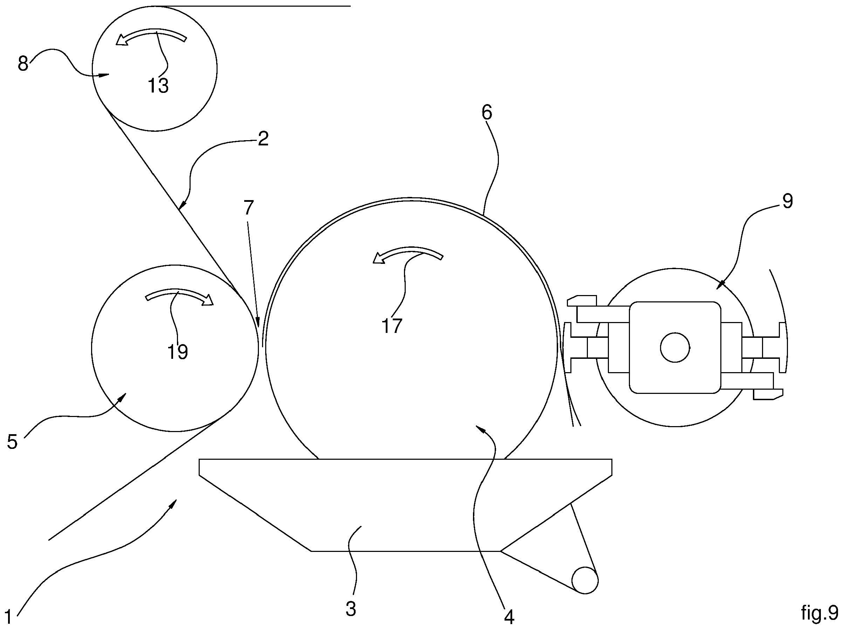

With reference to Figures 9-11, reference number 1 designates as a whole an apparatus for applying a pattern of glue onto a specific support provided for making sanitary articles.

As previously described, the apparatus 1 may be included in a machine for manufacturing sanitary articles, which may comprise multiple operative stations.

According to the invention, the apparatus 1 for applying a pattern of glue onto the support is configured for operating likewise a flexographic printing unit. It is to be noted that many construction details of the apparatus are not illustrated in the attached drawings, since they can be made according to techniques widely known in the technical field of reference.

The machine according to the invention may comprise only one apparatus 1 for applying a pattern of glue or the machine may have a number of such apparatus 1 that may be provided -for example- sequentially one after the other.

Figure 9 is a schematic cross-sectional view of an apparatus 1 showing the preferred arrangement of the main operating elements for obtaining an effective and versatile application of glue patterns onto a support provided for making sanitary articles.

The apparatus 1 comprises a frame (not illustrated), a plurality of rollers 4,5 for transferring a measured amount of glue and means for automatically continuously traveling a web 2 onto which the application of a specific pattern of glue is required. The rollers 4,5 are driven in rotation about parallel spaced-apart axes by means of drive motors (not illustrated) in connection with the actuation of said means for traveling the web 2, under the control of an electronic control unit. The rollers 4,5 may be driven to rotate respectively in the direction of the arrows 17,19. In one or more embodiments, the rollers 4,5 have independent different drive motors.

The means for traveling the web 2 may comprise a supply roller 8 - driven to rotate in the direction of the arrow 13 - (figure 9), provided for continuously fed the web 2 around an impression roller 5 which is described more in detail in the following.

According to the invention, the apparatus 1 comprises glue sourcing means 3,23 configured for providing glue to be transferred on the web 2.

In one or more embodiments, the glue sourcing means are a glue reservoir 3 provided for containing the glue which has to be taken and transferred to the support onto which a glue application is required. Preferably, the reservoir 3 is a hot glue reservoir configured for enabling the withdrawal of the required amounts of glue. The glue contained in the reservoir 3 may be liquid product (polyolefines or polyammide glue types) adapted to securely bond elastic films, woven webs, non-woven webs, polyethylene films, etc.

As illustrated in the embodiment of figure 10, the glue sourcing means may be a hot glue slot applicator 23 provided for applying precise glue amounts. In one or more embodiments, the glue applicator 23 may be of the spray type.

In one or more embodiments, the glue may be supplied via a wipe roller (for cold glue application), without departing from the scope of the present invention.

According to the invention, the apparatus 1 comprises a metering anilox roller 4 rotatably mounted for receiving glue and defining the required amount of glue to be transferred to the web 2.

In one or more embodiments, the anilox roller 4 may be provided partially embedded in the glue contained in the reservoir 3, in such a way that - when in rotation - is able to carry a glue layer 6 on its outer surface (figure 9).

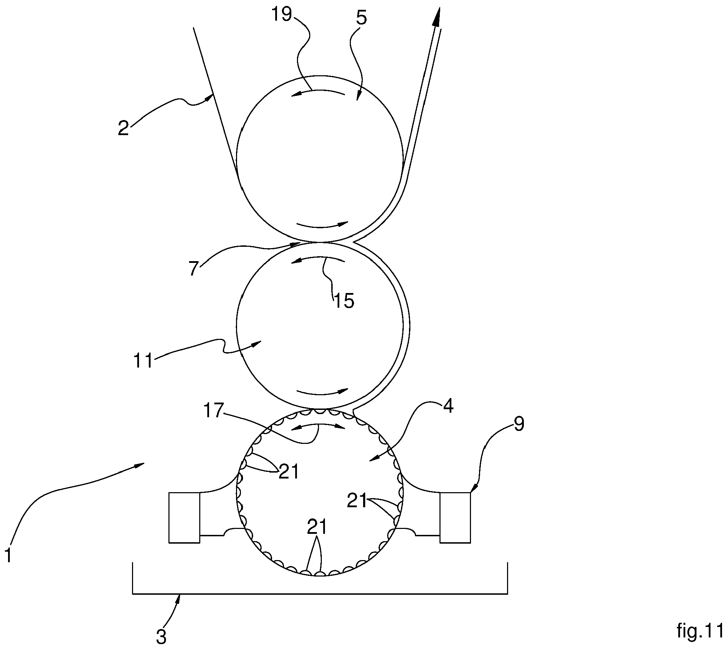

In one or more embodiments, the hot glue slot applicator 23 is provided for applying glue layers onto the outer lateral surface of the metering anilox roller 4 (figure 10). In this connection, the surface of the metering anilox roller 4 is engraved with multiple cells 21 having cavities (illustrated in figure 11), so as to provide a precise measured amount of glue delivered for the required glue application. The cells 21 may be provided with different dimensions and number of cave and shapes (for example spherical and/or pyramid shape). These differently engraved anilox surfaces can provide different glue amount and/or patterns to be applied onto the web 2. When installing a new apparatus 1, the cave/cells 21 dimension of the anilox roller 4 may be chosen which best results suits the majority of the gluing intended to be performed at that machine.

In one or more embodiments, the anilox roller 4 may be fully covered with edge engraved cave/cells 21, and the final glue shape is made via an impression roller 5 which comprises glue pattern shaping adapted to carry out the final glue shape on the web 2.

In one or more embodiments, the cave/cells 21 are provided only at specific areas of the outer lateral surface of the metering anilox roller 4, so as to provide directly the specific glue pattern onto the web 2, without the aid of further patterns formed on other rollers.

According to a further feature of the invention, the apparatus 1 comprises a blade assembly 9 configured to remove the glue in excess from the smooth non-engraved portions of the anilox roller 4, to create a uniform glue layer 6 to be transferred to the web 2 onto which a glue pattern is required. Typical materials for the blade assembly 9 are steel, carbon fiber or polymer.

The doctor blade assembly 9 may be provided as a single blade or dual blade (figure 9) configuration, to improve lifetime of the system. In a preferred configuration, the doctor blades are integrated in the hot glue vessel 3 close its side edges (figure 10). In order to keep the wear pattern as even as possible, and to help prevent any particles in the angle between blade and roller 4, the apparatus 1 is provided with oscillating/flexible blades. Important process variables are the blade pressure, the contact angle and the rotational position at which the blade wipes the anilox roller 4 and the pressure applied to the blade.

According to figures 9,10, the apparatus 1 comprises an impression roller 5 forming a nip 7 with the anilox roller 4, the web 2 passing through said nip 7. The impression roller 5 may be made of silicon or steel. The impression roller 5 may be configured with a flat surface or with specific shape provided for applying a specific glue pattern onto the web 2, using the glue transferred by the anilox roller 4. Therefore, the material to be glued passes between two rollers, the anilox roller 4 and the impression roller 5, the latter having the purpose of sliding and pressing the web 2 for transferring the glue layer 6 carried by the anilox roller 4, at a contact point between the web 2 and the glue layer 6.

According to the embodiment shown in figure 11, the apparatus 1 comprises a transfer roller 11 ("cliche") operatively interposed between the metering anilox roller 4 and the impression roller 5. In this case, the nip 7 is formed by the outer surfaces of the transfer roller 11 and the impression roller 5. The transfer roller 11 may be driven to rotate in the direction of the arrow 15.

The transfer roller 11 may comprise - in relief onto its outer surface - the pattern/shape intended to glue onto the web 2, so as to transfer the glue layer 6 carried by the anilox roller 4 to the web 2 and apply the intended pattern. In one or more embodiments, the intended pattern is obtained via a rubber sheet rolled onto the outer surface of transfer roller 11 or directly in monolithic cliche roll configuration. Also in this case, the metering anilox roller 4 may be provided with multiple cells 21 having cavities for determining the glue amount to be transferred to the transfer roller 11.

By actuating the rotation of the rollers 4,5,11, the glue moves from one roller to another, until it reaches the substrate to be glued, thanks to the different surface tension of the components with which it comes into contact.

The liquid glue detaches from the cells 21 of the anilox roller 4 and wets the surface of the next roller. In one or more embodiment, the cliche transfer roller 11 comprises additional micro-caves which receive the glue from the anilox roller 4, which transfer the micro drops of glue to the additional calibrated micro caves of the next roller (cliche), so as to define the best uniform distribution and resolution of the applied glue. Difference on surface tension comes from different type of raw materials and calibrated kiss pressure between cliche and anilox rolls gives the possibility to obtain a precise and flexible application of the glue which characterizes the machine according to the present invention.

Conventional adjustments and rollers direction (not shown) are provided for setting of the contact between the web 2 on the impression roller 5, the anilox roller 4 and the transfer roller 11.

In one or more embodiments, the apparatus 1 comprises a heated pressure chamber provided for containing hot melt to be transferred onto the web 2 via the rollers 4,5,11. The pressure chamber may be provided with a plurality of sealing elements for preventing leakage of the hot melt.

In these embodiments, the apparatus 1 comprises a plurality of heating elements - such as electrical resistances and/or infrared/UV lamps/sources - for heating the rollers 4,5,11 of the apparatus 1, in order to maintain the required conditions for the hot melt, after taking the hot melt with the anilox roller 4 and before the application onto the web 2. In case of hot melt application, the metering anilox roller 4 is configured for taking the hot melt from the heated pressure chamber. Naturally, the rollers 4,5,11 are made of different materials suitable for having significant wear resistance at high temperatures acceptable lifetime (for example hard silicon, tempered steel or ceramic coatings).

In these embodiments, the machine comprises further heating elements and thermoregulator elements located downstream the nip 7 through which the hot melt is applied, for maintaining the required conditions for the hot melt once applied onto the web 2.

In one or more embodiments, the apparatus 1 comprises a device for applying adhesive micro-powders onto the web 2, located downstream the glue application at said nip 7. The device is configured for applying the adhesive micro-powders onto the glue pattern applied, in order to further obtain glued joints between the sheets, extremely resistant to traction and frequent washing with hot water. In these embodiments, the apparatus 1 may comprise suction devices after powder application area for sucking any residual excess, located upstream the coupling area.

As compared to the prior art, the machine and method according to the present invention have the following advantages:
- applying a glue pattern with predetermined shape and grammature, at high speed;
- obtaining a precise application of a specific glue pattern on the area to be glued, without wasting glue sticking out from the target area;
- obtaining glue spots with circular shape;
- effectively gluing different materials, such as elastic film, woven web, non-woven web, polyethylene film, etc..

Of course, without prejudice to the principle of the invention, the details of construction and embodiments can be widely varied with respect to those described and illustrated, without thereby departing from the scope of the invention as defined by the claims that follow.

## Claims

1. Method for applying a glue pattern for making sanitary articles, comprising:
- automatically traveling a web (2) on which application of a glue pattern is required,
- actuating the rotation of a metering anilox roller (4) provided for transferring a glue layer (6),
- actuating the rotation of an impression roller (5) forming a nip (7) through which the web (2) travels,
- applying glue by means of glue sourcing means (3,23), so as to provide a glue layer (6) onto the outer surface of the anilox roller (4),
- transferring the glue layer (6) onto a receiving surface of the web (2) via said metering anilox roller (4).

2. The method of claim 1, wherein the step of applying glue by means of said glue sourcing means (3,6) comprises:
- providing a glue reservoir (3) associated with said anilox roller (4),
- drawing glue from said reservoir (3) by means of said anilox roller (4) driven in rotation, so as to provide a glue layer (6) onto the outer surface of the anilox roller (4).

3. The method of claim 1, wherein the step of applying glue by means of said glue sourcing means (3,23) comprises:
- providing a glue applicator (23) associated with said anilox roller (4),
- actuating said glue applicator (23) so as to provide a glue layer (6), onto the outer surface of the anilox roller (4) driven in rotation.

4. The method of claim 1, wherein the surface of the metering anilox roller (4) is engraved with multiple cells (21) having cavities, so as to provide a precise measured amount of glue to be transferred to the web (2).

5. The method of claim 4, wherein said multiple cells (21) are provided only at specific areas of the outer surface of the metering anilox roller (4), so as to provide the specific glue pattern onto the web (2) directly, without the aid of further patterns formed on other rollers (5,11).

6. The method of claim 4 or 5, comprising:
- providing a doctor blade assembly (9) associated with said anilox roller (4),
- removing the glue in excess from smooth non-engraved portions of the anilox roller (4), to create a uniform glue layer (6) to be transferred to the web (2).

7. The method of claim 1, wherein said nip (7) is formed between the outer surfaces of the metering anilox roller (4) and the impression roller (5).

8. The method of claim 1, comprising:
- providing a cliche transfer roller (11) operatively interposed between the metering anilox roller (4) and the impression roller (5), having in relief the pattern and shape of glue that must be applied on the web (2),
- forming said nip (7) between the outer surfaces of the transfer roller (11) and the impression roller (5),
- applying the glue pattern onto the web (2) via said cliche transfer roller (11).

9. The method of claim 1, comprising:
- providing a heated pressure chamber for containing hot-melt to be applied onto the web (2),
- providing a plurality of sealing elements for preventing leakage of the hot-melt,
- providing a plurality of heating elements for heating the rollers (4,5,11) of the machine, in order to maintain the required conditions for the hot-melt before the application onto the web (2).

10. The method of claim 9, comprising:
- providing further heating elements and thermoregulator elements located downstream the contact point of the hot melt with the web (2),
- maintaining the required conditions for the hot melt once applied onto the web (2) using said heating elements and thermoregulator elements.

11. Apparatus (1) for applying a glue pattern for making sanitary articles, wherein said apparatus (1) comprises:
- means for automatically continuously traveling a web (2) on which application of a pattern of glue is required,
- a metering anilox roller (4) provided for transferring a glue layer (6),
- glue sourcing means (3,23) configured for providing glue to be applied onto the outer surface of the metering anilox roller (4), and
- an impression roller (5) forming a nip (7) through which the web (2) may pass for receiving the glue transferred by the anilox roller (4),
- wherein, in operation, said apparatus (1) is configured for operating likewise a flexographic printing unit.

12. The apparatus (1) of claim 11, comprising a blade assembly (9) configured to remove the glue in excess from smooth non-engraved portions of the anilox roller (4), to create a uniform glue layer (6) to be transferred to the web (2).

13. The apparatus (1) according to claim 11, comprising a cliche transfer roller (11) operatively interposed between the metering anilox roller (4) and the impression roller (5), having in relief the pattern of glue that must be applied on the web (2), wherein said nip (7) is formed between the outer surfaces of the transfer roller (11) and the impression roller (5).

14. Method for manufacturing sanitary articles, comprising:
- providing a first continuous fabric layer (36) movable in a longitudinal direction (MD),
- providing an apparatus (1) for applying a glue pattern onto the fabric layer (36),
- automatically traveling said fabric layer (36),
- actuating the rotation of a metering anilox roller (4) provided for transferring a glue layer (6),
- actuating the rotation of an impression roller (5) forming a nip (7) through which the fabric layer (36) travels,
- applying glue by means of glue sourcing means (3,23), so as to provide a glue layer (6) onto the outer surface of the anilox roller (4),
- transferring the glue layer (6) onto a receiving surface of the fabric layer (36), via the metering anilox roller (4),
- providing a continuous absorbent tape (44) containing superabsorbent fibers, continuously moving in a longitudinal direction,
- transversely cutting said continuous absorbent tape (44) to form individual absorbent cores (28),
- spacing from each other in a longitudinal direction said individual absorbent cores (28),
- applying said absorbent cores (28) on said first continuous fabric layer (36) in longitudinally spaced positions over respective glue layers provided with said apparatus (1) for applying glue patterns.

15. An apparatus (32) for manufacturing sanitary articles, comprising:
- a first unwinding unit (34) configured for unwinding a first continuous fabric layer (36) from a first reel (38),
- a second unwinding unit (42) configured for unwinding a continuous washable absorbent tape (44) from a second reel (46),
- a first cut-and-slip unit (48) configured for transversally cutting the continuous washable absorbent tape (44) to form individual washable absorbent cores (28), wherein the first cut-and-slip unit (48) is configured for longitudinally spacing from each other the individual absorbent cores (28) and for applying the individual absorbent cores (28) on the first continuous fabric layer (36) in longitudinally spaced positions,
- a third unwinding unit (49) configured for unwinding a second continuous fabric layer (50) from a third reel (52),
- a second cut-and-slip unit (54) configured for transversally cutting the second continuous fabric layer (50) to form individual top layers (30), wherein the second cut-and-slip unit (54) is configured for longitudinally spacing from each other the individual top layers (30) and for applying the individual top layers (30) on respective absorbent cores (28) previously applied on the first continuous fabric layer (36), - an apparatus (1) for applying a glue pattern onto said first continuous fabric layer (36), comprising:
- a metering anilox roller (4) provided for transferring a glue layer (6)
- glue sourcing means (3,23) for providing glue to be applied onto the outer surface of the metering anilox roller (4), and
- an impression roller (5) forming a nip (7) through which the web (2) may pass, for receiving the glue transferred by the anilox roller (4),
- wherein, in operation, said apparatus (1) is configured for operating likewise a flexographic printing unit,
- said apparatus (1) being configured for permanently attaching by glue the individual absorbent cores (28) and the individual top layers (30) on the first continuous fabric layer (36).
